# EUROPEAN PATENT APPLICATION

(11) **EP 3 791 713 A1**
(43) Date of publication of application: **17.03.2021**
(21) Application number: 19196396.6
(22) Date of filing: 10.09.2019
(51) Int. Cl.: A01H 5/06, A01H 6/02, C12N 15/82, C12N 9/48, C12Q 1/6895

(54) **POLYMORPHISM FOR PATHOGEN RESISTANCE**

(71) Applicant: SESVanderHave N.V., 3300 Tienen (BE)
(72) Inventor: Hogenhout, Saskia Adriane, Norwich, NR4 7UH (GB); Mugford, Sam Thomas, Norwich, NR4 7UH (GB); Bernsdorff, Friederike, Norwich, NR1 4AH (GB); Drurey, Claire, Glasgow, G12 8TA (GB); Wilson-Lefevre, Christine, Norwich, NR9 3GD (GB); Kliot, Adi, Norwich, NR2 3JH (GB); Vanstraelen, Sigrid, 3300 Tienen (BE); Darracq, Aude, 3300 Tienen (BE)
(74) Representative: de Diesbach, Philippe

(57) **Abstract**

A method to select for plant resistance towards Hemipteran insects and/or towards Hemipteran-borne pathogens, the corresponding markers, mutants, isolated protein, recombinant constructs, and plant or plant part.

## Description

### Field of the invention

The present invention is in the field of agronomy and of resistance of sugar beet towards sucking (sap-feeding) insect (i.e. Hemipteran) and/or towards sucking insect-borne pathogens.

### Background of the invention

In crops, unacceptable proportions of the harvest may be lost due to pathogen infections.

Pathogens such as viruses, as well as prokaryotes, need vector for their propagation. Among these vectors are (sap-feeding) sucking insects (hemipteran).

Indeed, beyond the damage caused by the insect, the viruses (or other pathogens) they often carry, can result into unacceptable yield losses.

There is a tendency of a reduced use of pesticides, or even of a total ban of several classes of chemical compounds, including the neonicotinoid insecticides, even for a culture that does not flower under normal agricultural conditions, such as sugar beet.

As a consequence, there will be much more insects attacking plants, and also much more pathogens (viruses) propagated by these insects.

The generation of transgenic plants represents one way to tackle this problem. However, the commercialization of transgenic crops, even the crops transformed with constructs designed to exclusively target one or several pathogen(s), is difficult, especially for a crop like sugar beet.

Therefore, there exists a need to develop plants that are naturally resistant towards insects, or at least towards insect-borne pathogens, at least towards sap-feeding insects (i.e. Hemipteran) and/or sap-feeding insect-borne pathogens.

### Summary of the invention

A first aspect of the present invention is a method for selecting a *Beta vulgaris* plant resistant towards Hemipteran and/or Hemipteran-borne pathogens comprising the step of measuring the presence of a polymorphism in SEQ ID NO:1 or in SEQ ID NO:2 in the said *Beta vulgaris* plant and of selecting a *Beta vulgaris* plant having the said polymorphism, and preferably a further step of selecting a *Beta vulgaris* plant having a polymorphism in SEQ ID NO:1 or in SEQ ID NO:2 which results into a loss of interaction with SEQ ID NO:4 (and/or with the orthologs thereof) and/or which results into a resistance towards Hemipteran and/or towards (at least one) Hemipteran-borne pathogen(s).

Hence, preferably, this polymorphism in SEQ ID NO:1 or in SEQ ID NO:2 causes the loss of functional interaction with Mp10 (SEQ ID NO:4) and ortholog proteins of hemipteran (such as SEQ ID NO:10).

The preferred polymorphism is at the amino acids (residues) 20 (instead of a G), 25 (instead of a V), 27 (instead of a A), 38 (instead of a A), 45 (instead of a V), 52 (instead of a T), 60 (instead of a R), 72 (instead of a V), 109 (instead of a N), 175 (instead of a M), 177 (instead of a V), 188 (instead of a P), 206 (instead of a F) and/or 208 (instead of a N) of SEQ ID NO:2, being more preferably a D at position 45, a I at position 52, a T at position 72, a I (or a L) at position 109, a K at position 175, a I at position 177, a L at position 188, a Y at position 206 and/or a K at position 208, more preferably being a I or a L at position 109 of SEQ ID NO:2 (Y206 is already present in this sequence).

A related aspect of the present invention is the corresponding nucleotide probe(s) for the detection of a polymorphism in SEQ ID NO:2, preferably wherein the polymorphism is at the codon encoding the amino acid (residues) 20, 25, 27, 38, 45, 52, 60, 72, 109, 175, 177, 188, 206 and/or 208 of SEQ ID NO:2, being preferably V45D, V72T, N109I (or N109L), F206Y and/or N208K , respectively, and more preferably at the codon encoding asparagine 109 of SEQ ID NO:2.

Another related aspect of the present invention is a *Beta vulgaris* plant, plant part or plant cell having a polymorphism in SEQ ID NO:1 or in SEQ ID NO:2, preferably the polymorphism depicted here above, possibly homozygote for this polymorphism, and more preferably having on both alleles a polymorphism in SEQ ID NO:1 and/or in SEQ ID NO:2.

Advantageously, this plant part is a treated *Beta vulgaris* seed, not comprising neonicotinoid insecticide.

Possibly, this *Beta vulgaris* plant is genetically altered.

Another related aspect of the present invention is the use of this *Beta vulgaris* plant (or plant part, possibly a seed not comprising neonicotinoid insecticide) for growing in a field affected by Hemipteran and/or by Hemipteran-borne pathogens, possibly selected from the group consisting of Beet mild yellowing virus (BMYV), Beet yellowing virus (BYV), Beet Curly Top virus, Beet chlorosis virus, Beet Leaf yellowing virus, Beet Western Yellowing virus, Syndrome Basse Richesse' (SBR) proteobacterium and SBR phytoplasma.

Another related aspect of the present invention is an isolated AMSH2 variant protein sharing at least 95% of identity with SEQ ID NO: 2 over at least 50 consecutive amino acids, and harboring preferably a polymorphism at the residues 20, 25, 27, 38, 45, 52, 60, 72, 109, 175, 177, 188, 206 and/or 208 of SEQ ID NO:2, being more preferably the variant selected from the group consisting of SEQ ID NO:5, SEQ ID NO:6, SEQ ID NO:7, SEQ ID NO:8 and SEQ ID NO:9.

Another related aspect of the present invention is an isolated nucleic acid molecule comprising a sequence sharing at least 95% of identity, but less than 100% of identity with SEQ ID NO:1 and preferably encoding the mutant (AMSH2 variant depicted here above).

Another related aspect of the present invention is a vector comprising this isolated nucleic acid.

Another related aspect of the present invention is a transgenic plant or (plant) cell comprising this isolated nucleic acid molecule or this vector.

Still another related aspect of the present invention is a method to select a sugar beet plant expressing an additional factor providing synergistic resistance towards Hemipteran and/or Hemipteran-borne pathogens comprising to generate a sugar beet population expressing the AMSH2 mutant (depicted here above), to test this generated population for a resistance towards this Hemipteran and/or towards this Hemipteran-borne pathogens and to identify the (non-AMSH2) factor providing this synergistic resistance and to select the identified plants having increased resistance towards Hemipteran and/or Hemipteran-borne pathogens.

### Detailed description of the invention

The inventors have identified that BvAMSH2 protein (SEQ ID NO: 2; the protein encoded by XM_010684251.2) interacts with sap-feeding insects (Hemipteran, i.e. plant-feeding Hemipteran, such as *Sternorrhyncha* (comprising aphids) and/or *Auchenorryncha* (comprising hoppers)), and/or sap-feeding insects effectors, at least aphids (and planthoppers or leafhoppers) factors (such as Mp10; SEQ ID NO:4), and that this interaction hampers the plant response towards the insect (Hemipteran) infection.

AMSH2 (SEQ ID NO:3) knock-out is lethal in *Arabidopsis;* however, the inventors have found specific AtAMSH2 mutants which are not lethal, whereas they are no longer recognized by insects' effectors. In addition, AMSH2 (SEQ ID NO:2) knock-out is not lethal in sugar beet, providing more flexibility in this crop.

Although insect viability is not necessarily markedly affected by such AMSH2 mutants, the inventors have found that AMSH2 mutant plants are more resistant towards pathogen infections borne by sap-feeding insects (Hemipteran), also in synergy with other factors providing resistance towards sap-feeding insects (Hemipteran) and/or towards the pathogen (viruses, microorganism) carried by them.

Therefore, a first aspect of the present invention is a method for selecting a sugar beet (*Beta vulgaris*) plant being resistant to hemipteran insects and/or to hemipteran-borne pathogens (virus, microorganism), comprising the identification of a polymorphism in SEQ ID NO:1 or in SEQ ID NO:2.

The inventors have found that the factor of *Myzus persicae* responsible of the interaction with AMSH2 (SEQ ID NO:4; herein also referred to as Mp10; a member of the OS-D superfamily) is conserved in all the Hemipteran species analyzed so far, even in planthoppers (SEQ ID NO:10).

In the context of the present invention, "Hemipteran" (or "Hemiptera") preferably refers to a taxonomic order of insects comprising aphids and planthoppers.

Important Hemipteran insects acting as vector for pathogens (virus or microorganism) are selected from the group consisting of *Pentastiridius sp.* (Cixiidae), beet leafhopper *(Circulifer tenellus), Myzus persicae,* and *Aphis fabae,* wherein *Circulifer tenellus, Myzus persicae,* and *Aphis fabae* are the most important so far.

In the context of the present invention, *"Beta vulgaris"* preferably refers to its normal taxonomic denomination. Hence "Beta *vulgaris"* encompasses *"Beta vulgaris vulgaris"* (including the *Altissima* Group, the *Cicla* Group, the *Flavescens* Group and the *Crassa* Group) and *"Beta vulgaris maritima",* as well as any other subspecies or variety belonging to the *Beta vulgaris* species.

Any modification in the gene (including the promoter and the 3' and 5' UTR regions) encoding SEQ ID NO:1 or SEQ ID NO:2 are encompassed by the present invention, including amino acid substitution(s), addition(s) or deletion(s) of one or of several amino acid(s), as well as the loss of functionality of SEQ ID NO:1 or of SEQ ID NO:2, due to a lower expression level (e.g. a mutation in the non-coding regions, such as in the promoter, the 3' UTR and/or in the 5'UTR) or to a premature STOP codon.

The preferred modifications in SEQ ID NO:1 or SEQ ID NO:2 result into amino acid substitutions, and more preferably at amino acids G20, V25, A27, A38, V45, V72, and/or N109 SEQ ID NO:2.

The most preferred mutations in SEQ ID NO:1 or SEQ ID NO:2 are located at amino acids V45, V72 and N109, and still more preferably at amino acid N109.

Suitable mutations are selected from the group consisting of V45D, V72T and N109I (or N109L).

One substitution at a key residue, e.g. N109I, is enough to confer resistance to Hemipterans and/or Hemipteran-borne pathogens (micro-organisms or virus). However, the present invention also covers a stack of several mutations.

One convenient way to identify suitable mutations in SEQ ID NO:1 or SEQ ID NO:2 is an *in vitro* test for measuring the loss of interaction with its Hemipteran-binding factor, such as SEQ ID NO:4, SEQ ID NO:10 and the orthologs thereof.

In the context of the present invention, an ortholog of SEQ ID NO:4 and/or of SEQ ID NO:10 is an Hemipteran factor being member of the OS-D superfamily and having a significant homology with SEQ ID NO:4 and/or SEQ ID NO:10, as well as the capacity to bind *in vitro* to (non-mutated) SEQ ID NO:1 or SEQ ID NO:2, or possibly to SEQ ID NO:2 harboring a V72I and/or a Y206F mutation.

For instance a "significant homology" means at least 20% (preferably at least 30%, more preferably at least 40%) of identity (or of homology) with SEQ ID NO:4 and/or with SEQ ID NO:10 over the full-length of SEQ ID NO:4 or of SEQ ID NO:10, for instance, measured using BLASTp algorithm (e.g. using a BLOSUM 62 matrix, a "word size" of 6, and a "gap cost (Existence; Extension)" of 11; 1; when BLASTp is used with these parameters, "homology" is equated as the proportion of identical residues plus the proportion of the residues marked as "positive" by the BLASTp algorithm). Other bio-informatic tools can be used for determining a significant homology.

In the context of the present invention, the *in vitro* binding between (i) SEQ ID NO:4 and/or the orthologs thereof and (ii) AMSH2 (SEQ ID NO:2) or the AMSH2 variants having a substitution of one or of several amino acids in SEQ ID NO:2 is measured (quantified) preferably by yeast two hybrid system, by pull-down or by co-immunoprecipitation. A possible way to quantify such *in vitro* binding is performed by comparison between (i) the *in vitro* binding between SEQ ID NO:2 (or possibly to SEQ ID NO:2 harboring a V72I and/or a Y206F mutation) and SEQ ID NO:4 (positive control) and (ii) the *in vitro* binding between SEQ ID NO:8 or SEQ ID NO:9, and SEQ ID NO:4 (negative control) .

Another possible method to determine the loss of interaction between BvAMSH2 mutants and its sap-feeding insect effector (such as SEQ ID NO:4 or SEQ ID NO:10) is to transform *Nicotiana benthamiana* leaf discs expressing the sap-feeding insect effector (such as SEQ ID NO:4 or SEQ ID NO:10) with the BvAMSH2 mutant, to induce an immune response (e.g. ROS production), and to measure whether the BvAMSH2 mutant allows for an increased response over the WT BvAMSH2 (for more details, see below the Example 3).

A related aspect of the present invention is the (isolated) gene (or cDNA) encoding an AMSH2 variant, resulting into a lower expression of SEQ ID NO:2, or into the expression of a premature STOP codon in SEQ ID NO:2 or into a substitution of one or of several amino acid(s) in SEQ ID NO:2, as depicted here above (also referred to as AMSH2 variant). Preferably, such AMSH2 variant is a (an isolated) nucleic acid molecule comprising a sequence sharing at least 95% of identity, but less than 100% of identity with SEQ ID NO:1, and encoding the full-length (e.g. at least 200 amino acids) SEQ ID NO:2 with a substitution of one or of several amino acid(s) therein, preferably at the residues 20, 25, 27, 38, 45, 52, 60, 72, 109, 175, 177, 188, 206 and/or 208 of SEQ ID NO:2, as mentioned here above.

Another related aspect of the present invention is nucleotide probe(s) for the detection of the presence of a polymorphism in SEQ ID NO:2, preferably wherein the polymorphism is at the codon encoding the amino acid (residues) 20, 25, 27, 38, 45, 52, 60, 72, 109, 175, 177, 188, 206 and/or 208 of SEQ ID NO:2, being preferably V45D, V72T, N109I (or N109L) and/or F206Y, respectively, more preferably at the codon for N109I (or N109L).

Such probes, preferably, span over the above-mentioned polymorphism. However, by aligning the chromosomal region encoding WT BvAMSH2 (SEQ ID NO:1) and a mutant BvAMSH2, equivalent probes can be designed using other polymorphisms, even polymorphisms at the nucleotide level and not resulting into an amino acid change in SEQ ID NO:2, or chromosomal polymorphism upstream and/or downstream of SEQ ID NO:1, since these additional polymorphisms also reflect the presence of the mutant AMSH2, even if not necessarily directly causing a reduced interaction with Mp10 (SEQ ID NO:4) and the orthologs thereof.

Another related aspect of the present invention is a *Beta vulgaris* plant, plant part or plant cell harboring a polymorphism in SEQ ID NO:1 or in SEQ ID NO:2. Preferably, such plant is not obtained by essentially biological methods. The polymorphism can be at the residues 20, 25, 27, 38, 45, 52, 60, 72, 109, 175, 177, 188, 206 and/or 208 of SEQ ID NO:2.

Preferably, the present invention relates to isolated cell or plant part harboring a polymorphism in SEQ ID NO:1 or in SEQ ID NO:2.

Preferably, this *Beta vulgaris* plant is harboring a polymorphism in SEQ ID NO:1 or in SEQ ID NO:2, but not the V45D or V72T or Y206F polymorphisms.

More preferably, this *Beta vulgaris* plant comprises (expresses the protein of) SEQ ID NO:8 or SEQ ID NO:9.

Preferably, the present invention relates to a treated sugar beet seed harboring a polymorphism in SEQ ID NO:1 or in SEQ ID NO:2, wherein this treated seed does not comprise systemic insecticides, such as the neonicotinoid insecticide.

By "treated sugar beet seed", it is preferably meant any seed treatment involving a (one or several, e.g. two or three) technical step under controlled conditions (humidity, temperature and/or duration). Among the usual seed treatments are the coating, the pelleting and the priming of the seeds.

"Coating of a seed", in the context of the present invention, preferably refers to the addition of an external layer to the seed or to the pellet comprising a seed (see below). Such layer can comprise a coloring agent, and one or several active chemical agent(s), preferably selected from the group consisting of minerals/cations (B, K, Ca, Mg, Cu, Fe, Co, Ni, Mo, Mn, Zn, Ag), anions (phosphorous-based or sulfur-based), oligomers of biomolecules, synthetic polymers, herbicides, fungicides, or insecticides (not systemic insecticides).

In the context of the present invention, "a sugar beet seed pellet" preferably refers to an (artificially) embedded sugar beet seed in a polymer matrix. The pellet can comprise active chemical agent(s), as in the coating (see above). The seed pellet can further comprise one or several exogenous (i.e. not naturally present in the seed) microorganism species or spores for one or several exogenous microorganisms.

In the context of the present invention, a "primed sugar beet seed" preferably refers to a seed that has undergone a treatment under controlled humid conditions (humidity, temperature and/or time), then a controlled drying stage. Alternatively, or in addition, the primed seeds are in late Phase II, or even in Phase III of the germination process. Hence, the priming of seeds allows to speed up, and to increase the uniformity of, the germination process upon sowing.

Still another related aspect of the present invention is the use of such a *Beta vulgaris* plant harboring a polymorphism in SEQ ID NO:1 or in SEQ ID NO:2 for growing in a field affected by Hemipteran and/or by Hemipteran-borne pathogens.

Preferably, such polymorphism in SEQ ID NO:1 or in SEQ ID NO:2 results into the loss of interaction (for instance measured *in vitro*) with SEQ ID NO:4 and/or in SEQ ID NO:10 and/or the orthologs thereof.

The preferred field affected by Hemipteran and/or by Hemipteran-borne pathogens is a field where insecticides, preferably systemic insecticides, such as neonicotinoid insecticides are not used.

Preferably, this field is infested, or is susceptible to be infested, by hemipteran-borne pathogens, more preferably selected from the group consisting of Beet mild yellowing virus (BMYV), Beet yellowing virus (BYV), Beet Curly Top virus, Beet chlorosis virus, Beet Leaf yellowing virus, Beet Western Yellowing virus, the Syndrome Basse Richesse' (SBR) proteobacterium and the SBR phytoplasma.

Another related aspect of the present invention is an isolated AMSH2 variant protein sharing at least 95% of identity with SEQ ID NO: 2 over at least 50 consecutive amino acids. The preferred AMSH2 variants have a polymorphism in SEQ ID NO:2 at one or several of the residues 20, 25, 27, 38, 45, 52, 60, 72, 109, 175, 177, 188, 206 and/or 208 of SEQ ID NO:2, being preferably V45D, V72T, N109I (or N109L) (and/or F206Y), being more preferably N109I.

Such preferred variants are selected from the group consisting of SEQ ID NO:5, SEQ ID NO:6, SEQ ID NO:7, SEQ ID NO:8, SEQ ID NO:9, SEQ ID NO:11 and SEQ ID NO:12.

The AMSH2 variant protein can be tested *in vitro* for a binding to SEQ ID NO:4 and/or SEQ ID NO:10 and/or to the ortholog(s) thereof.

Another related aspect of the present invention is an isolated nucleic acid encoding this variant protein, as well as a vector comprising such isolated nucleic acid molecule.

Another related aspect of the present invention is a transgenic plant or (plant) cell comprising this isolated nucleic acid molecule and/or vector.

Having understood the present general resistance mechanism, the inventors have found other minor resistance factor(s) that synergize(s) with it, whereas such minor additional resistance factors are not easily identified on their own.

Another related aspect of the present invention is therefore a method to identify an additional sugar beet factor providing a synergistic resistance towards Hemipteran and/or Hemipteran-borne pathogens comprising to generate a sugar beet population expressing the AMSH2 mutant of the present invention, to test the said generated population for a resistance towards the said Hemipteran and/or towards the said Hemipteran-borne pathogens and to identify the (non-AMSH2) factor providing the said synergistic resistance.

### Brief description of the figures

Figure 1 shows plant immune response to Mp10 (SEQ ID NO:4; grey bars; black bars, no Mp10) in function of the AMSH2 sequence.
Figure 2 shows Yeast two Hybrid test based on Mp10 (SEQ ID NO:4), and on different mutations in AMSH2 (SEQ ID NO:2).

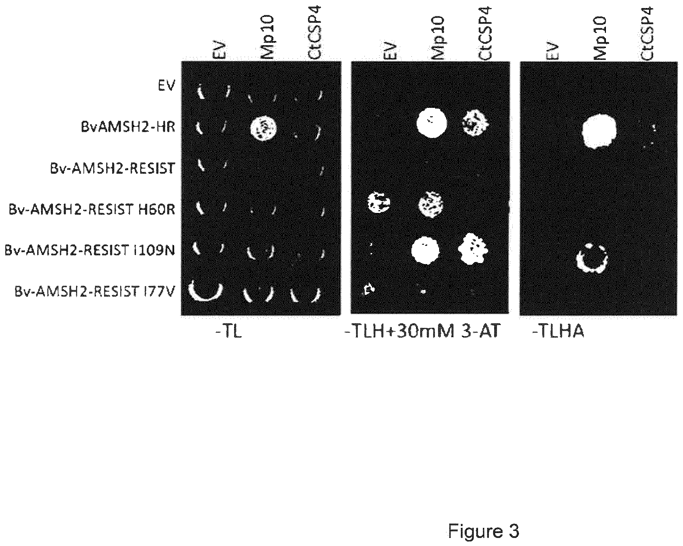

Figure 3 shows Yeast two Hybrid test based on Mp10 (SEQ ID NO:4) or on the hopper ortholog (CtCSP4), and on different revert-mutations in AMSH2 (SEQ ID NO:2).
Figure 4 shows the resistance in field conditions, induced by the N109I mutation in AMSH2 against Curly top virus using a scoring scale between 1, not-damaged plants, to 9: plants completely destroyed by Curly top virus.

### Examples

### Example 1. Validation of Mp10

In the genesis of the invention, the inventors have performed *in silico* analysis and have singled out a few conserved proteins involved in insect feeding that might be associated with infectivity.

Then the inventors have found that a plant tissue infected with aphids (*Myzus persicae;* Green Peach Aphid) bearing silenced Mp10 (SEQ ID NO:4) produces much more reactive oxygen: this Mp10 factor impairs the plant immune response.

Example 2. Identification of plant factors interacting with Mp10.

The inventors have developed a yeast two-hybrid system to identify putative plant binding partners.

AMSH2 from different plant species, including *Arabidopsis thaliana* (Seq ID NO:3), *Beta vulgaris* (SEQ ID NO:2) and *Nicotiana sp.* were shown to interact with Mp10 (SEQ ID NO:4).

### Example 3. ROS response of AMSH2 mutants

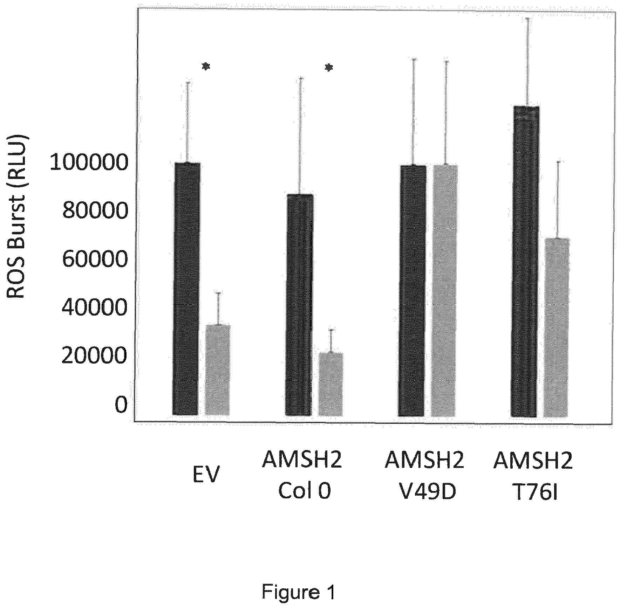

The inventors have measured the production of Reactive Oxygen Species (ROS, a marker of plant immune response) by tissues expressing mutated AMSH2 together with Mp10: Mp10 no longer blocks ROS response in the case of non-interacting AMSH2 mutants (Figure 1). More into details, *Nicotiana benthamiana* leaf discs transiently expressing Mp10 show a suppressed reactive oxygen species (ROS) burst in response to treatment with the bacterial elicitor-flg22, compared to control leaf discs (EV, compare black and grey bars). In leaf discs coexpressing non-interacting alleles of AMSH2 (V49D or T76I) together with Mp10; Mp10 is no longer able to suppress the flg22-induced ROS burst (right).

### Example 4. Screening of AMSH2 mutants not interacting with Mp10

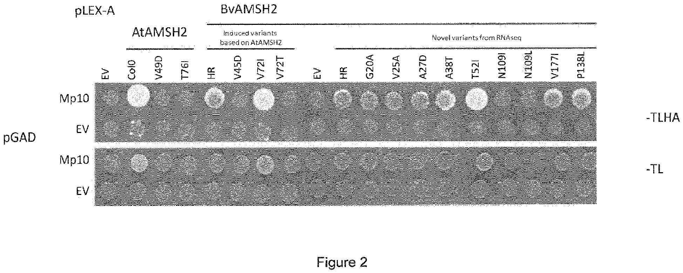

The inventors then obtained AMSH2 mutants (mutagen, screening of naturally-occurring plants, edited or lab-made constructs) and screened them in yeast-two hybrid with Mp10. Among them, in *Arabidopsis,* V49D and T76I showed a markedly reduced interaction (Figures 1 and 2). In *Beta vulgaris,* V45D and V72T showed a markedly reduced interaction, whereas V72I showed an increased interaction (Figure 2). The mutation of the asparagine at position 109 (into I or L) abolished the interaction with Mp10.

Then, the inventors have developed the same test, starting with a mutant, non-interacting, AMSH2, and reverting back one residue as in WT AMSH2 (SEQ ID NO:2). Here, the inventors have measured the binding of the AMSH2 variants towards Mp10 (SEQ ID NO:4), as well as towards its hopper ortholog CtCSP4; SEQ ID NO:10). The back-mutation reversing the amino acid residue at position 109 into the original Asparagine (N) allowed a positive signal. The inventors conclude that an Asparagine at such position is a key for the interaction with the Hemipteran Mp10 and the orthologs thereof; hence sugar beet mutants at this position represent ideal candidates for resistance towards Hemipteran and/or Hemipteran-borne pathogens.

### Example 5. Effect of AMSH2 mutants on aphid viability

The inventors then measured the aphid viability in *Arabidopsis* AtAMSH2 mutants. Surprisingly, the inventors did not notice any major effect on aphid viability.

### Example 6. Screen for more AMSH2 mutants

The inventors then obtained a wider set of AMSH2 mutants of SEQ ID NO:2 (sugar beet; *Beta vulgaris*) and cloned the mutants for yeast two-hybrid experiments.

Among them, again, N109I and N109L showed a marked reduction of the interaction with Mp10.

### Example 7. Validation in field trials

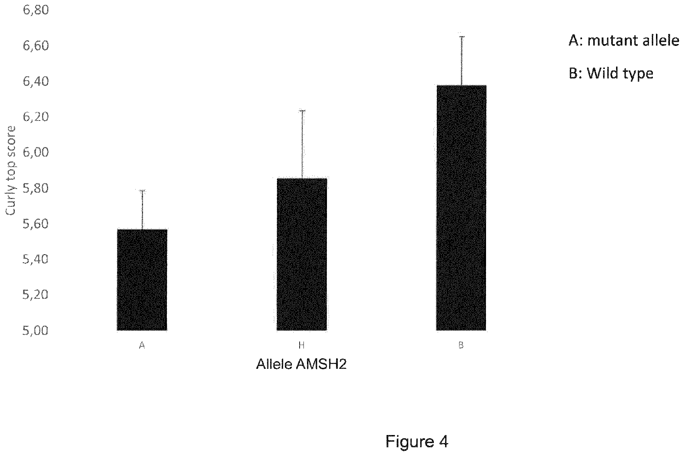

Sugar beet plants with heterozygote and homozygote N109I mutation in SEQ ID NO:2 (i.e. SEQ ID NO:8) have been tested in field trials for resistance towards (i) Curly top virus and (ii) Beet mild yellowing virus. Beet Curly top were loaded on the beet leafhopper, before challenge of sugar beet (Figure 4), or Beet mild yellowing virus were loaded on green peach aphid before challenge of sugar beet. The plants with the homozygote N109I mutation (condition A) have a marked reduction of curly top symptoms, and the heterozygote plants at this position (condition H) have an intermediate phenotype.

The inventors therefore conclude that mutations in AMSH2 so as to keep a functional protein but sufficient to abolish its binding to Mp10, provides a useful resistance towards sap-feeding insect (hemipteran; aphids and/or planthoppers or leafhoppers) -borne pathogens.

## Claims

1. A method for selecting a *Beta vulgaris* plant resistant towards Hemipteran and/or Hemipteran-borne pathogens comprising the steps of measuring the presence of a polymorphism in SEQ ID NO:1 or in SEQ ID NO:2 in the said *Beta vulgaris* plant and of selecting a *Beta vulgaris* plant having the said polymorphism.

2. The method of claim 1, wherein the polymorphism in SEQ ID NO:1 or in SEQ ID NO:2 causes the loss of functional interaction with Mp10 (SEQ ID NO:4) and/or with ortholog Hemipteran proteins.

3. The method of claim 1 or of claim 2, wherein the polymorphism is at the amino acid position(s) selected from the group consisting of position 20, 25, 27, 38, 45, 52, 60, 72, 109, 175, 177, 188 and/or 208 of SEQ ID NO:2, being preferably selected from the group consisting of a D at position 45, a I at position 52, a T at position 72, a I or a L at position 109, a K at position 175, a I at position 177, a L at position 188 and/or a K at position 208, more preferably being a I or a L at position 109.

4. Nucleotide probes for the detection of a polymorphism in SEQ ID NO:2, preferably wherein the polymorphism is at the codon encoding the amino acid position 20, 25, 27, 38, 45, 52, 60, 72, 109, 175, 177, 188 and/or 208 of SEQ ID NO:2.

5. The probes of claim 4, wherein the polymorphism is at the codon encoding asparagine 109 of SEQ ID NO:2.

6. A *Beta vulgaris* plant, plant part or plant cell having a polymorphism in SEQ ID NO:1 or in SEQ ID NO:2, preferably the polymorphism according to any one of the preceding claims, and more preferably being homozygote for the said polymorphism, and/or having on both alleles a polymorphism in SEQ ID NO:1 and/or in SEQ ID NO: 2 according to any one of the preceding claims.

7. The plant part of claim 6 being a treated *Beta vulgaris* seed, not comprising neonicotinoid insecticide.

8. The plant part of claim 6 or 7 being genetically altered.

9. Use of the plant or of the plant part according to any one of the preceding claims 6 to 8 for growing in a field affected by Hemipteran and/or by Hemipteran-borne pathogens.

10. Use of claim 9, wherein the Hemipteran-borne pathogen is one or several pathogen(s) selected from the group consisting of Beet mild yellowing virus (BMYV), Beet yellowing virus (BYV), Beet Curly Top virus, Beet chlorosis virus, Beet Leaf yellowing virus, Beet Western Yellowing virus, 'Syndrome Basse Richesse' (SBR) proteobacterium and SBR phytoplasma.

11. An isolated AMSH2 variant protein sharing at least 95% of identity with SEQ ID NO:2 over at least 50 consecutive amino acids, and harboring preferably a polymorphism at the residues 20, 25, 27, 38, 45, 52, 60, 72, 109, 175, 177, 188 and/or 208 of SEQ ID NO:2, being more preferably the variant selected from the group consisting of SEQ ID NO:5, SEQ ID NO:6, SEQ ID NO:7, SEQ ID NO:8 and SEQ ID NO:9.

12. An isolated nucleic acid molecule comprising a sequence sharing at least 95% of identity, but less than 100% of identity with SEQ ID NO:1 and preferably encoding the variant of claim 11.

13. A vector comprising the isolated nucleic acid of claim 11 or of claim 12.

14. A transgenic plant or (plant) cell comprising the protein of claim 11 or the nucleic acid molecule of claim 12 and/or the vector of claim 13.

15. A method to select a sugar beet plant expressing an additional factor providing synergistic resistance towards Hemipteran and/or Hemipteran-borne pathogens comprising to generate a sugar beet population expressing the AMSH2 mutant of claim 11 or of claim 12, to test the said generated population for a resistance towards the said Hemipteran and/or towards the said Hemipteran-borne pathogens and to identify the (non-AMSH2) factor providing the said synergistic resistance and to select the identified plants having increased resistance towards Hemipteran and/or Hemipteran-borne pathogens.


SEQUENCE LISTING

<110>  SES VanderHave NV

<120>  POLYMORPHISM FOR PATHOGEN RESISTANCE

<130>  SESS0019AEP

<160>  12

<170>  PatentIn version 3.5

<210>  1
<211>  1161
<212>  DNA
<213>  Beta vulgaris

<220>
<221>  CDS
<222>  (229)..(888)
<223>  Bv AMSH2

<400>  1
aaaaaaaaaa aaaaaacaat ctttccccca aagtatcatc agaattgcaa ttactaccgt      60

tattctccac ttcttccctc tccttcaaat tagcaattta tttgttcaca aacatacaga     120

aattatcgta tatagtcact tattttaaag caatttcacc tcttttcctc cctcatcctt     180

ctcaaacatg caggaataag acgtacgaac atcaaaggga atagaaat atg gtc act     237
                                                     Met Val Thr
                                                     1

cat tac cct tct gca cca atc tct tgc acg gag gtt cca cct aat gct     285
His Tyr Pro Ser Ala Pro Ile Ser Cys Thr Glu Val Pro Pro Asn Ala
    5                   10                  15

gga caa gtt tca caa gtg aca gct gca gat tca cga tat gca tat tca     333
Gly Gln Val Ser Gln Val Thr Ala Ala Asp Ser Arg Tyr Ala Tyr Ser
20                  25                  30                  35

gac tct gcg att cat gtt ctt aaa aat gta cat att tca gct caa tta     381
Asp Ser Ala Ile His Val Leu Lys Asn Val His Ile Ser Ala Gln Leu
                40                  45                  50

aca gat gat ttt ctt gaa ctt gcc cgc aac aac acc aac aac gat ctc     429
Thr Asp Asp Phe Leu Glu Leu Ala Arg Asn Asn Thr Asn Asn Asp Leu
            55                  60                  65

gag aca tgt ggg gtt ctt ggt gct tcc ctc aaa gaa gag aca ttc tat     477
Glu Thr Cys Gly Val Leu Gly Ala Ser Leu Lys Glu Glu Thr Phe Tyr
        70                  75                  80

gct aca acg tta ata ata ccg aag cag aaa tca act tcc agc tca tgt     525
Ala Thr Thr Leu Ile Ile Pro Lys Gln Lys Ser Thr Ser Ser Ser Cys
    85                  90                  95

cag gca tta agg gaa gag gag atc ttt aac ata cag aat gat cgg tcc     573
Gln Ala Leu Arg Glu Glu Glu Ile Phe Asn Ile Gln Asn Asp Arg Ser
100                 105                 110                 115

cta ttt ccc gta gga tgg atc cat aca cat cca tct caa agt tgt ttc     621

Leu Phe Pro Val Gly Trp Ile His Thr His Pro Ser Gln Ser Cys Phe
                120                 125                 130

atg tca tcg ata gat ctg cac acg caa tat cct tat cag gta atg gtg       669
Met Ser Ser Ile Asp Leu His Thr Gln Tyr Pro Tyr Gln Val Met Val
            135                 140                 145

ccg gag gct atc gcc att gtc gtg gct ccc act gat aag aca agg aat       717
Pro Glu Ala Ile Ala Ile Val Val Ala Pro Thr Asp Lys Thr Arg Asn
        150                 155                 160

tat ggt ata ttc cgg ctg acg gat cct agt gga atg aac gtt gtt aag       765
Tyr Gly Ile Phe Arg Leu Thr Asp Pro Ser Gly Met Asn Val Val Lys
    165                 170                 175

gaa tgc gaa gag aac ggg ttc cac cct cac cgg gat tcc acc gac ggt       813
Glu Cys Glu Glu Asn Gly Phe His Pro His Arg Asp Ser Thr Asp Gly
180                 185                 190                 195

agg ccg ttg tat gaa caa tgc tca cat gtt tat atc aac cca aac cta       861
Arg Pro Leu Tyr Glu Gln Cys Ser His Val Tyr Ile Asn Pro Asn Leu
                200                 205                 210

aga ttt gaa atc ttt gat ctt cgt tga tatgactgaa catactgctt             908
Arg Phe Glu Ile Phe Asp Leu Arg
            215

tgtttccaca tttgaagaca attgaggtac atttctttgc aatcatgcat gtaaagctta     968

gtacggctta gcatcagtag tcagttccaa cttccaaatg tagtacaaac tcttgttgtg    1028

cacaaggtta catagatcca tctcctgtag attgttaaaa aagtgtaaat gcttctaatt    1088

gtccactgct caatatgaat ataggattaa cttaaaagga ctaaataaaa tttgatgtat    1148

tttttctttt gca                                                       1161

<210>  2
<211>  219
<212>  PRT
<213>  Beta vulgaris

<400>  2

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Asn Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  3
<211>  223
<212>  PRT
<213>  Arabidopsis thaliana

<400>  3

Met Val Thr Leu Ser Ser Pro Ser Pro Ser Leu Ser Cys Val Glu Asn
1               5                   10                  15

Val Thr Cys Lys Ser Ser His Val Ser Arg Val Leu Ile Ser Gly Thr
            20                  25                  30

Asp Asn Ile Asn His Gly Glu Ser Ser Glu Ala Lys Ile Leu Arg Asp
        35                  40                  45

Val His Ile Ser Glu Arg Leu Leu Glu Asp Phe Thr Glu Leu Ala Arg
    50                  55                  60

Glu Asn Thr Glu Lys Asp Leu Glu Thr Cys Gly Thr Leu Ala Ala Phe
65                  70                  75                  80

Leu Glu Arg Gly Ile Phe Tyr Val Thr Thr Leu Ile Ile Pro Lys Gln
                85                  90                  95

Glu Ser Thr Ser Asn Ser Cys Gln Ala Met Asn Glu Val Glu Val Phe
            100                 105                 110

Ser Ile Gln Asn Glu Arg Glu Leu Tyr Pro Val Gly Trp Ile His Thr
        115                 120                 125

His Pro Ser Gln Gly Cys Phe Met Ser Ser Val Asp Leu His Thr His
    130                 135                 140

Tyr Ser Tyr Gln Val Met Val Pro Glu Ala Phe Ala Ile Val Val Ala
145                 150                 155                 160

Pro Thr Asp Ser Ser Lys Ser Tyr Gly Ile Phe Lys Leu Thr Asp Pro
                165                 170                 175

Gly Gly Met Glu Val Leu Arg Gly Cys Ser Glu Thr Gly Phe His Pro
            180                 185                 190

His Lys Glu Pro Glu Asp Gly Asn Pro Val Tyr Glu His Cys Ser Asn
        195                 200                 205

Val Tyr Lys Asn Ser Asn Leu Arg Phe Glu Ile Phe Asp Leu Arg
    210                 215                 220

<210>  4
<211>  153
<212>  PRT
<213>  Myzus persicae

<220>
<221>  MISC_FEATURE
<222>  (1)..(9)
<223>  Alternative ATG

<400>  4

Met Phe Ser Thr Gln Ser Gln Ala Met Asp Ser Arg Ile Ala Val Val
1               5                   10                  15

Cys Val Val Leu Ala Val Phe Ala Val Asp Gln Thr Val Gly Ala Pro
            20                  25                  30

Gln Lys Asp Ala Val Ala Ala Ser Gly Pro Ala Tyr Thr Thr Lys Tyr
        35                  40                  45

Asp His Ile Asp Ile Asp Gln Val Leu Gly Ser Lys Arg Leu Val Asn
    50                  55                  60

Ser Tyr Val Gln Cys Leu Leu Asp Lys Lys Pro Cys Thr Pro Glu Gly
65                  70                  75                  80

Ala Glu Leu Arg Lys Ile Leu Pro Asp Ala Leu Lys Thr Gln Cys Val
                85                  90                  95

Lys Cys Asn Ala Thr Gln Lys Asn Ala Ala Leu Lys Val Val Asp Arg
            100                 105                 110

Leu Gln Arg Asp Tyr Asp Lys Glu Trp Lys Gln Leu Leu Asp Lys Trp
        115                 120                 125

Asp Pro Lys Arg Glu Tyr Phe Gln Lys Phe Gln Gln Phe Leu Ala Glu
    130                 135                 140

Glu Lys Lys Lys Gly Val Val Lys Phe
145                 150

<210>  5
<211>  219
<212>  PRT
<213>  Beta vulgaris

<220>
<221>  MISC_FEATURE
<222>  (45)..(45)
<223>  Mutation in non-interacting varient

<220>
<221>  MISC_FEATURE
<222>  (72)..(72)
<223>  Mutation in non interacting varient

<220>
<221>  MISC_FEATURE
<222>  (109)..(109)
<223>  Mutation in non-interacting varient

<220>
<221>  MISC_FEATURE
<222>  (175)..(175)
<223>  Mutation in non-interacting varient

<220>
<221>  MISC_FEATURE
<222>  (206)..(206)
<223>  Mutation (not a F) in non-interacting varient

<220>
<221>  MISC_FEATURE
<222>  (208)..(208)
<223>  Mutation in non-interacting varient

<400>  5

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Asn Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  6
<211>  219
<212>  PRT
<213>  Beta vulgaris

<220>
<221>  MISC_FEATURE
<223>  Mutant V45D

<400>  6

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Asp His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Asn Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  7
<211>  219
<212>  PRT
<213>  Beta vulgaris

<220>
<221>  MISC_FEATURE
<223>  Mutant V72T

<400>  7

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Thr Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Asn Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  8
<211>  219
<212>  PRT
<213>  Beta vulgaris

<220>
<221>  MISC_FEATURE
<223>  Mutant N109I

<400>  8

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Ile Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser

180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  9
<211>  219
<212>  PRT
<213>  Beta vulgaris

<220>
<221>  MISC_FEATURE
<223>  Mutant N109L

<400>  9

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Leu Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  10
<211>  145
<212>  PRT
<213>  Aphis fabae

<400>  10

Met Asp Ser Arg Ile Ala Val Val Cys Val Val Leu Ala Val Phe Ala
1               5                   10                  15

Val Asp Gln Thr Val Gly Ala Pro Gln Lys Asp Ala Val Ala Val Gly
            20                  25                  30

Gly Pro Ala Tyr Thr Thr Lys Tyr Asp His Ile Asp Ile Asp Gln Val
        35                  40                  45

Leu Ala Ser Lys Arg Leu Val Asn Ser Tyr Val Gln Cys Leu Leu Asp
    50                  55                  60

Lys Lys Pro Cys Thr Pro Glu Gly Ala Glu Leu Arg Lys Ile Leu Pro
65                  70                  75                  80

Asp Ala Leu Lys Thr Gln Cys Ala Lys Cys Asn Ala Thr Gln Lys Asn
                85                  90                  95

Ala Ala Leu Lys Val Val Asp Arg Leu Gln Lys Asp Tyr Asp Ala Glu
            100                 105                 110

Trp Lys Gln Leu Leu Asp Lys Trp Asp Pro Lys Arg Glu His Phe Gln
        115                 120                 125

Lys Phe Gln Gln Phe Leu Ala Glu Glu Lys Lys Lys Gly Phe Thr Lys
    130                 135                 140

Phe
145

<210>  11
<211>  219
<212>  PRT
<213>  Beta vulgaris

<220>
<221>  MISC_FEATURE
<222>  (175)..(175)
<223>  M175K mutation

<400>  11

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Asn Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Lys Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Asn
        195                 200                 205

Pro Asn Leu Arg Phe Glu Ile Phe Asp Leu Arg
    210                 215

<210>  12
<211>  219
<212>  PRT
<213>  Beta vulgaris

<220>
<221>  MISC_FEATURE
<222>  (208)..(208)
<223>  N208K mutation

<400>  12

Met Val Thr His Tyr Pro Ser Ala Pro Ile Ser Cys Thr Glu Val Pro
1               5                   10                  15

Pro Asn Ala Gly Gln Val Ser Gln Val Thr Ala Ala Asp Ser Arg Tyr
            20                  25                  30

Ala Tyr Ser Asp Ser Ala Ile His Val Leu Lys Asn Val His Ile Ser
        35                  40                  45

Ala Gln Leu Thr Asp Asp Phe Leu Glu Leu Ala Arg Asn Asn Thr Asn
    50                  55                  60

Asn Asp Leu Glu Thr Cys Gly Val Leu Gly Ala Ser Leu Lys Glu Glu
65                  70                  75                  80

Thr Phe Tyr Ala Thr Thr Leu Ile Ile Pro Lys Gln Lys Ser Thr Ser
                85                  90                  95

Ser Ser Cys Gln Ala Leu Arg Glu Glu Glu Ile Phe Asn Ile Gln Asn
            100                 105                 110

Asp Arg Ser Leu Phe Pro Val Gly Trp Ile His Thr His Pro Ser Gln
        115                 120                 125

Ser Cys Phe Met Ser Ser Ile Asp Leu His Thr Gln Tyr Pro Tyr Gln
    130                 135                 140

Val Met Val Pro Glu Ala Ile Ala Ile Val Val Ala Pro Thr Asp Lys
145                 150                 155                 160

Thr Arg Asn Tyr Gly Ile Phe Arg Leu Thr Asp Pro Ser Gly Met Asn
                165                 170                 175

Val Val Lys Glu Cys Glu Glu Asn Gly Phe His Pro His Arg Asp Ser
            180                 185                 190

Thr Asp Gly Arg Pro Leu Tyr Glu Gln Cys Ser His Val Tyr Ile Lys
        195                 200                 205

Pro Asn Leu Arg Phe Glu Ile Phe Asp Leu Arg
    210                 215